# FASCICULE DE BREVET EUROPEEN

(11) **EP 0 659 175 B1**
(45) Date de publication et mention de la délivrance du brevet: **13.08.1997**
(21) Numéro de dépôt: 93919420.5
(22) Date de dépôt: 06.09.1993
(51) Int. Cl.: C07C 309/24, C07K 5/06

(54) **DERIVES D'(AMINO-3 PHENYL)-1 ETHANESULFONATE D'ALKYLAMMONIUM OPTIQUEMENT ACTIFS, LEUR PREPARATION ET LEUR UTILISATION**
OPTISCH AKTIVE ALKYLAMMONIUM 1-(3-AMINOPHENYL) ETHANSULFONAT-DERIVATE, IHRE HERSTELLUNG SOWIE IHRE VERWENDUNG
OPTICALLY ACTIVE ALKYLAMMONIUM (AMINO-3 PHENYL)-1 ETHANESULFONATE DERIVATIVES, PREPARATION AND USE THEREOF

(30) Priorité: 11.09.1992 FR 9210840
(43) Date de publication de la demande: 28.06.1995
(73) Titulaire: RHONE-POULENC RORER S.A., 92160 Antony (FR)
(72) Inventeur: GUYON, Claude, F-94100 Saint-Maur-des-Fossés (FR)
(74) Mandataire: Savina, Jacques
(86) Numéro de dépôt international: FR9300849
(87) Numéro de publication internationale: WO9406760

(56) Documents cités:
- WO-A-91/13907
- FR-A- 2 132 818

## Description

La présente invention concerne les isomères lévogyres des (amino-3 phényl)-1 éthanesulfonate d'alkylammonium de formule: 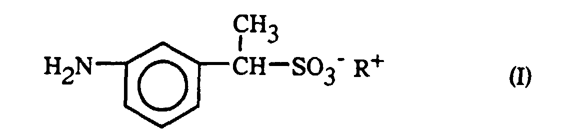 dans laquelle R⁺ représente un reste tétraalkylammonium ou trialkylphénylalkyl ammonium, leur préparation et leur utilisation comme intermédiaires pour la préparation de composés utiles comme antagonistes de la cholécystokinine (CCK) et de la gastrine.

Dans les définitions précédentes, et celles qui suivent, les radicaux alkyle contiennent 1 à 4 atomes de carbone en chaîne droite ou ramifiée. Ils représentent de préférence le radical n-butyl.

Les composés de formule (I) (isomères lévogyres) peuvent être préparés selon le procédé suivant :
a) action de sulfite d'un métal alcalin sur le (bromo-1 éthyl)-1 nitro-3 benzène-(RS) et, si nécessaire, passage au sel de potassium pour obtenir le (nitro-3 phényl)-1 éthanesulfonate de potassium-(RS),
b) transformation du sel de potassium obtenu en a) en (nitro-3 phényl)-1 éthanesulfonate de benzylquininium mélange des formes A et B, séparation de la forme A et récupération d'un mélange enrichi en forme B,
c) conversion du sel de benzylquininium enrichi en forme B obtenu en b) en (nitro-3 phényl)-1 éthanesulfonate de potassium puis en (nitro-3 phényl)-1 éthanesulfonate de phénylglycinol forme B et enfin en (nitro-3 phényl)-1 éthanesulfonate de tétraalkylammonium ou trialkylphénylalkylammonium (isomère lévogyre),
d) réduction du produit obtenu en c) en composé de formule (I) (isomère lévogyre).

Il est particulièrement avantageux d'effectuer l'étape a) en solution aqueuse, à une température comprise entre 50 °C et 100°C et de préférence à 80°C.

Le sulfite de métal alcalin est de préférence le sulfite de sodium ou de potassium.

Il est préférable pour récupérer le produit de le transformer en sel de tétraalkylammonium ou de trialkylphénylammonium, l'isoler puis de le transformer en sel de potassium.

L'étape b) s'effectue de préférence au moyen d'un halogénure de N-benzylquininium et de préférence le chlorure, en présence de dihydrogénophosphate de potassium, en milieu aqueux et à une température comprise entre 10 °C et 30 °C et en particulier à une température voisine de 20°C.

Le sel de potassium de l'étape c) est obtenu par action de nonafluorobutanesulfonate de potassium, au sein d'un solvant inerte tel que l'acétone, à une température comprise entre 10°C et 30 °C et de préférence à 20°C.

Le sel de phénylglycinol de l'étape c) est obtenu par action de (R)-(-) phénylglycinol, en présence d'un acide tel que l'acide chlorhydrique, en milieu aqueux, à une température comprise entre 10°C et 30°C et de préférence à 20°C.

Le sel de phénylglycinol est ensuite transformé en composé de formule (I) au moyen d'un sel de tétraalkylammonium ou de trialkylphénylalkylammonium et de préférence les hydrogénosulfates, en milieu aqueux, à une température comprise entre 10°C et 30°C et de préférence à 20°C.

La réduction de l'étape d) s'effectue généralement sous pression d'hydrogène, en présence d'un catalyseur d'hydrogénation tel que le palladium, au sein d'un solvant inerte tel qu'un alcool (éthanol par exemple), à une température voisine de 25°C. On utilise de préférence une pression d'hydrogène de 100 kPa.

Les composés de formule (I) (isomères lévogyres) sont particulièrement intéressants comme intermédiaires pour la préparation des énantiomères des composés de formule : 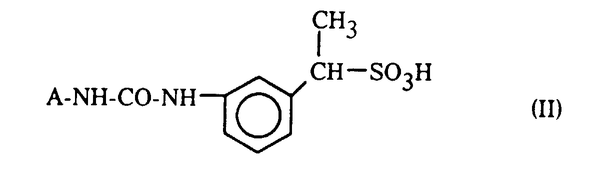 et leurs sels.

Dans la formule (II), A représente :
A) un reste -CH₂-CO-NR₁R₂ dans lequel R₁ représente un radical (a) phényle éventuellement substitué par un ou plusieurs substituants choisis parmi les atomes d'halogène et les radicaux alkyle, alcoxy, hydroxy polyfluoroalkyle, nitro, alkylthio, alcoxycarbonyle, carboxy, acylamino, méthylènedioxy, polyfluoroalcoxy, trifluorométhylthio, phénoxy, phényl, benzyle, phénylamino et CONR₃R₄, identiques ou différents, représentent un atome d'hydrogène, un radical alkyle, phényle (éventuellement substitué par un ou plusieurs substituants choisis parmi les atomes d'halogène et les radicaux alkyle, alcoxy et alkylthio), indanyle, cycloalkylalkyle, cycloalkyle, phénylalkyle ou bien R₃ et R₄ forment ensemble avec l'atome d'azote auquel ils sont rattachés un hétérocycle mono ou polycyclique saturé ou insaturé contenant 4 à 9 atomes de carbone et un ou plusieurs hétéroatomes (O,N,S) et éventuellement substitué par un plusieurs radicaux alkyle, alcoxy, alcoxycarbonyle, dialkylcarbamoyle, phényle ou en combinaison avec un atome de carbone de l'hétérocycle par un cycle spiromonocyclique à 4 ou 5 chaînons et contenant éventuellement un ou plusieurs hétéroatomes (O,S,N) ; b) pyridyle, c) isoquinolyle, d) quinolyle, e) quinoxalinyle (ces hétérocycles étant éventuellement substitués par un ou plusieurs substituants choisis parmi les radicaux alkyle, phényle et les atomes d'halogène), f) alkyle, g) phénylalkyle, h) naphtyle, i) tétrahydro-5,6,7,8 naphtyle, j) tétrahydro-1,2,3,4 naphtyle, k) alcoxycarbonylalkyle ou l) cycloalkyle. R₂ représente une chaîne -CH(R₅)-CO-R₆ dans laquelle R₅ représente un atome d'hydrogène ou un radical alkyle, alcoxycarbonyle ou phényle (éventuellement substitué par un ou plusieurs substituants choisis parmi les atomes d'halogène et les radicaux alkyle, alcoxy, alkylthio, nitro et amino) et R₆ représente un radical alcoxy, cycloalkyloxy (éventuellement substitué par au moins un radical alkyle), cycloalkylalkyloxy, phénylalkyloxy, polyfluoroaLkyloxy cinnamyloxy et -NR₃R₄.
B) un reste de formule : 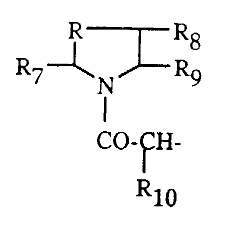 dans laquelle:
   - soit R représente un radical méthylène, éthylène, SO, SO₂, CHOH ou un atome de soufre, R₇ représente un radical pyridyle éventuellement substitué par un ou plusieurs radicaux alkyle, furyle éventuellement substitué par un ou plusieurs radicaux alkyle, thiènyle éventuellement substitué par un ou plusieurs radicaux alkyle, quinolyle éventuellement substitué par un ou plusieurs radicaux alkyle, naphtyle éventuellement substitué par un ou plusieurs radicaux alkyle, indolyle éventuellement substitué par un ou plusieurs radicaux alkyle ou phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-NR₁₁R_{12,} -NH-CO-CH₃, trifluorométhyle ou trifluorométhoxy et R₈ représente un atome d'hydrogène,
   - soit R représente un radical méthylène, R₇ représente un atome d'hydrogène et R₈ représente un radical phényle,
   - soit R représente un radical CHR₁₃, R₇ et R₈ représentent chacun un atome d'hydrogène,
   - R₉ représente un radical alcoxycarbonyle, cycloalkyloxycarbonyle, cycloalkylalkyloxycubonyle, -CONR₁₄R₁₅ ou phényle éventuellement substitué par un ou plusieurs substituants choisis parmi les radicaux alkyle, alcoxy ou hydroxy,
   - R₁₀ représente un atome d'hydrogène ou un radical alkyle,
   - R₁₃ représente un radical phényle,
   - R₁₁ représente un atome d'hydrogène ou un radical alkyle, phénylalkyle ou phényle éventuellement substitué par un ou plusieurs substituants choisis parmi les atomes d'halogène et les radicaux alkyle, alcoxy et alkylthio,
   - R₁₂ représente un radical alkyle, phénylalkyle ou phényle éventuellement substitué par un ou plusieurs substituants choisis parmi les atomes d'halogène et les radicaux alkyle, alcoxy et alkylthio,
   ou bien R₁₁ et R₁₂ forment avec l'atome d'azote auquel ils sont rattachés un hétérocycle mono ou polycyclique saturé ou insaturé contenant 4 à 9 atomes de carbone et un ou plusieurs hétéroatomes (O, N) et éventuellement substitué par un ou plusieurs radicaux alkyle,
   - R₁₄ représente un atome d'hydrogène ou un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
   - R₁₅ représente un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
   ou bien R₁₄ et R₁₅ forment ensemble avec l'atome d'azote auquel ils sont rattachés un hétérocycle mono ou polycyclique saturé ou insaturé contenant 4 à 9 atomes de carbone et un ou plusieurs hétéroatomes (O, N, S) et éventuellement substitué par un ou plusieurs radicaux alkyle,
C) un reste de formule : 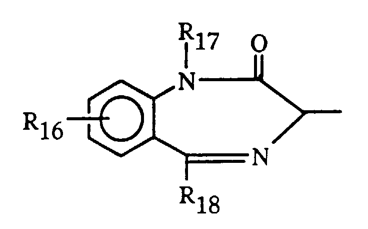 dans laquelle :
   - R₁₆ représente un atome d'hydrogène ou d'halogène ou un radical alkyle, alkylthio, nitro, hydroxy ou cyano,
   - R₁₇ représente un radical alkyle ou un reste -CH(R₅)-CO-R₆,
   - R₁₈ représente un radical pyridyle ou phényle éventuellement substitué par un ou plusieurs substituants choisis parmi les atomes d'halogène et les radicaux alkyle, alcoxy, hydroxy, carboxy et nitro.

Dans les définitions des composés de formule (II), les radicaux alkyle et alcoxy et les portions alkyle et alcoxy contiennent de préférence 1 à 4 atomes de carbone en chaîne droite ou ramifiée, les radicaux et portions cycloalkyle 3 à 6 atomes de carbone et les radicaux acyle 2 à 4 atomes de carbone.

Ces composés sont décrits dans les demandes de brevets WO 91/12264, WO 91/13907, WO 91/13874, FR 9108675 et FR 9112481 comme antagonistes de la cholécystokinine et de la gastrine.

Les composés de formule (II) peuvent être préparés à partir des composés de formule (I) en opérant de la manière suivante :

on fait agir un composé de formule (I) sur un dérivé de formule : 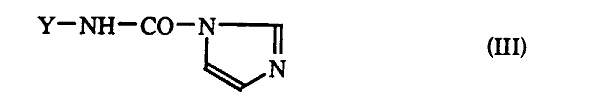 dans laquelle Y a les mêmes significations que dans la formule (II).

Cette réaction s'effectue généralement au sein d'un solvant inerte tel que le tétrahydrofuranne, le diméthylformamide, un solvant chloré, un solvant aromatique (benzène, toluène par exemple), à une température comprise entre 20°C et la température d'ébullition du solvant

Les dérivés de formule (III) peuvent être préparés selon les procédés décrits dans les demandes de brevets WO 91/12264, WO 91/13907, WO 91/13874, FR 9108675 et FR 9112481.

### EXEMPLE 1

a) (nitro-3 phényl)-1 éthanesulfonate de potassium-(RS) :
A une solution de 20,8 g de sulfite de sodium dans 260 cm³ d'eau on ajoute 25,3 g de (bromo-1 éthyl)-1 nitro-3 benzène-(RS). Le mélange réactionnel est agité à 80°C pendant 5 heures, refroidi à environ 25°C et coulé dans 2,5 litres d'une solution aqueuse de dihydrogénophosphate de potassium 0,5 M. On ajoute 40 g d'hydrogénosulfate de tétra-n-butyl ammonium. Le mélange est extrait par 3 fois 500 cm³de chlorure de méthylène. Les phases organiques réunies sont lavées par 2 fois 500 cm³ d'eau, séchées sur sulfate de magnésium et concentrées à sec sous pression réduite (2,7 kPa) à 40°C. L'huile obtenue est dissoute dans 65 cm³ d'acétone et on ajoute 34 g de nonafluorobutanesulfonate de potassium en solution dans 75 cm³ d'acétone. Le produit insoluble est séparé par filtration, lavé par 3 fois 50 cm³ d'oxyde de diisopropyle et séché à l'air. On obtient ainsi 22,4 g de (nitro-3 phényl)-1 éthanesulfonate de potassium-(RS), fondant à une température supérieure à 260°C et utilisé tel quel dans les synthèses ultérieures.
Spectre de R.M.N. : (200 MHz; DMSO d)

| δ(ppm) : | |
|---|---|
| 1,50 | [d, J=7 Hz, 3H: -CH(CH₃)-] |
| 3,93 | [q, J=7 Hz, 1H: -CH(CH₃)-] |
| 7,59 | [t, J=8 Hz, 1H: -C₆H₄(-H5)] |
| 7,83 | [d, J=8 Hz, 1H: -C₆H₄(-H6)] |
| 8,10 | [d large,J=8 Hz, 1H: -C₆H₄(-H4)] |
| 8,26 | [s large, 1H: -C₆H₄(-H2)]. |

Le (bromo-1 éthyl)-1 nitro-3 benzène-(RS) peut être préparé selon la méthode décrite par E. FELDER et Coll, J. Med. Chem., 13, 559 (1970).
b) (nitro-3 phényl)-1 éthanesulfonate de N-benzylquininium, mélange des formes A et B :
A une solution de 17,2 g de (nitro-3 phényl)-1 éthanesulfonate de potassium-(RS) dans 400 cm³ d'eau, on ajoute 87 g de dihydrogénophosphate de potassium et 32,4 g de chlorure de N-benzyl quininium. Le mélange est extrait par 2 fois 300 cm³ de chlorure de méthylène. Les phases organiques réunies sont lavées par 2 fois 200 cm³ d'eau, séchées sur sulfate de magnésium et concentrées à sec sous pression réduite (2,7 kPa)) à 40°C. La meringue obtenue est dissoute dans 120 cm³ de propanol-2 à reflux. Après refroidissement, les cristaux sont séparés par filtration, lavés par 2 fois 15 cm³ de propanol-2. On obtient, après 2 recristallisations dans 350 puis 500 cm³ de propanol-2, 15,6 g de (nitro-3 phényl)-1 éthanesulfonate de N-benzylquininium, forme A, fondant à environ 110°C. [α]_{D}²⁰ = -151,3° ± 1,5 (C = 1,009 %; Méthanol). Les solutions propanoliques sont réunies et concentrées à sec sous pression réduite (2,7 kPa) à 45°C. On obtient ainsi 25,0 g de (nitro-3 phényl)-1 éthanesulfonate de N-benzylquininium, mélange des formes A et B (environ 15/85 en moles).
c) (nitro-3 phényl)-1 éthanesulfonate de tétra-n-butyl ammonium, isomère lévogyre :
A une solution de 10,5 g de (nitro-3 phényl)-1 éthanesulfonate de N-benzylquininium, mélange des formes A et B (environ 15/85 en moles), dans 16 cm³ d'acétone, on ajoute 5,2 g de nonafluorobutanesulfonate de potassium en solution dans 12 cm³ d'acétone. Le produit insoluble est séparé par filtration, puis dissous dans 9 cm³ d'eau. On ajoute 8,4 cm³ d'une solution aqueuse d'acide chlorhydrique 1 N et 1,15 g de (R)-(-)-phénylglycinol. La solution obtenue est concentrée à sec sous pression réduite (2,7 kPa) à 50°C. Le résidu obtenu est extrait par 3 fois 15 cm³ d'acétonitrile à reflux. Les phases organiques sont réunies, et concentrées à environ 7 cm³ ; après refroidissement, les cristaux sont séparés par filtration et dissous dans 7,5 cm³ d'une solution aqueuse de soude 1 N. La solution obtenue est lavée par 8 fois 25 cm³ d'oxyde de diéthyle, puis on ajoute 60 cm³ d'une solution aqueuse de dihydrogénophosphate de potassium 0,5 M et 2,3 g d'hydrogénosulfate de tétra-n-butyl ammonium. Le mélange est extrait par 3 fois 30 cm³ de chlorure de méthylène. Les phases organiques réunies sont séchées sur sulfate de magnésium et concentrées à sec sous pression réduite (2,7 kPa) à 40°C. On obtient ainsi 2,8 g de (nitro-3 phényl)-1 éthanesulfonate de tétra-n-butylammonium, isomère lévogyre, sous forme d'une huile utilisée telle quelle dans les synthèses ultérieures.
d) (amino-3 phényl)-1 éthanesulfonate de tétra-n-butyl ammonium, isomère lévogyre:
A une solution de 2,8 g de (nitro-3 phényl)-1 éthanesulfonate de tétra-n-butyl ammonium, isomère lévogyre, dans 50 cm³ d'éthanol, on ajoute 0,2 g de palladium sur noir à 5 %. La suspension est agitée pendant 2 heures à une température voisine de 25°C sous atmosphère d'hydrogène (100 kPa). Le catalyseur est séparé par filtration et le filtrat est concentré à sec sous pression réduite (2,7 kPa) à 40°C. On obtient ainsi 2,8 g d'(amino-3 phényl)-1 éthanesulfonate de tétra-n-butyl ammonium, isomère lévogyre, sous forme d'une huile utilisée telle quelle dans les synthèses ultérieures.
Spectre de R.M.N. : (200 MHz; CDCl₃, forme B)

| δ(ppm) : | |
|---|---|
| 0,98 | (t, J=7 Hz, 12H: -CH₃ du butyl) |
| de 1,25 à 1,65 | (mt, 16H: -CH₂-CH₂-CH₃ du butyl) |
| 1,65 | (d, J=7 Hz, 3H: -CH(CH₃)) |
| 2,95 | (mf, 2H: -NH₂) |
| 3,15 | (mt, 8H: >N-(CH₂-) du butyl) |
| 3,91 | (q, J=7 Hz, 1H: -CH(CH₃)-) |
| 6,50 | (d, large, J=8 Hz, 1H: -C₆H₄ (H4)) |
| 6,85 | (d, J=8 Hz, 1H: -C₆H₄ (H6)) |
| 6,86 | (s large, 1H: -C₆H₄ (H2)) |
| 7,00 | (t, J=8 Hz, 1H: -C₆H₄ (H5)) |

### EXEMPLE D'APPLICATION

A une solution de 2,1 g d'{[(imidazolyl-1) carboxamido]-2 N-(méthoxy-3 phényl) acétamido)-2 N-méthyl N-phényl-acétamide dans 130 cm³ de toluène, on ajoute 2,7 g d'(amino-3 phényl)-1 éthanesulfonate de tétra-n-butyl ammonium, isomère lévogyre. Le mélange réactionnel est agité à reflux pendant 5 heures, puis concentré à sec sous pression réduite (2,7 kPa) à 45°C. Le résidu est dissous dans 100 cm³ de chlorure de méthylène et la solution obtenue est lavée par 50 cm³ d'une solution aqueuse d'acide chlorhydrique 2N, puis par 2 fois 50 cm³ d'eau. La phase organique est séchée sur sulfate de magnésium et concentrée à sec sous pression réduite (2,7 kPa) à 40°C. Le produit brut obtenu est agité 30 minutes dans 50 cm³ d'oxyde de diisopropyle. Le produit insoluble est séparé par filtration puis dissous dans 6 cm³ d'acétone. On ajoute 1,2 g de nonafluorobutanesulfonate de potassium en solution dans 3 cm³ d'acétone puis 5 cm³ d'oxyde de diisopropyle. La gomme insoluble est séparée, puis agitée pendant 1 heure dans 12 cm³ d'un mélange d'acétone et d'oxyde de diisopropyle (50/50 en volumes). Le produit insoluble est séparé par filtration, lavé par 2 fois 5 cm³ d'un mélange d'acétone et d'oxyde de diisopropyle (50/50 en volumes) puis 4 fois 5 cm³ d'oxyde de diisopropyle et séché à l'air. On obtient ainsi 1,55 g de (-)-{{[N-(méthoxy-3 phényl) N-(N-méthyl N-phényl-carbamoylméthyl) carbamoylméthyl]-3 uréido}-3 phényl}-1 éthanesulfonate de potassium fondant à environ 180°C. [α]_{D}²⁰ = - 5,0°± 0,5 (C = 0,888 %; Méthanol).
Spectre de R.M.N.: (300 MHz; DMSO d6: forme (-))

| δ(ppm): | |
|---|---|
| 1,43 | [d,J=7Hz,3H: -CH(CH₃)-] |
| 3,18 | [s large, 3H; -N(CH₃)-] |
| 3,60 | [mt, 1H: -CH(CH₃)-] |
| 3,65 | [d large, J=5Hz, 2H: -CO(CH₂)NH-] |
| 3,79 | (s, 3H: -OCH₃) |
| 4,09 | [mf, 2H: -CO(CH₂)N<] |
| 6,28 | [t large, J=5Hz, 1H: -NH-] |
| 6,86 | [d,J=7.5 Hz, 1H: -C₆H₄(-H4) du N-(Methoxy-3 phényl)] |
| de 6,95 à 7,15 | (mt, 4H: aromatiques) |
| 7,17 | (s large, 1H:-C₆H₄(-H2) du N-(méthoxy-3 phényl)] |
| de 7,30 à 7,50 | (mt, 11H: aromatiques). |
| 8,80 | (s large, 1H: -CO-NH-Ar). |

L'{[(imidazolyl-1) carboxamido]-2 N-(méthoxy-3 phényl) acétamido}-2 N-méthyl N-phényl-acétamide peut être préparé de la manière suivante : à une solution de 3,0 g de N,N'-dimidazolecarbonyle dans 30 cm³ de tétrahydrofuranne anhydre, on ajoute une solution de 3,1 g d'[amino-2 N-(méthoxy-3 phényl) acétamido]-2 N-méthyl N-phényl-acétamide dans 30 cm³ de tétrahydrofuranne anhydre. La solution est agitée pendant 16 heures à une température voisine de 25°C puis concentrée à sec sous pression réduite (2,7 kPa) à 40°C. Le résidu est dissous dans 50 cm³ d'acétate d'éthyle et la solution obtenue est lavée par 4 fois 30 cm³ d'eau. La phase organique est séchée sur sulfate de magnésium puis concentrée à sec sous pression réduite (2,7 kPa) à 40°C. On obtient, après recristallisation dans l'acétate d'éthyle, 3,5 g d'{[(imidazolyl-1) carboxamido]-2 N-(méthoxy-3 phényl) acétamido}-2 N-méthyl N-phényl-acétamide fondant à 146°C

L'[amino-2 N-(méthoxy-3 phényl) acétamido]-2 N-méthyl N-phényl-acétamide peut être préparé de la manière suivante : à une solution de 5,5 g de [N-(méthoxy-3 phényl) phtalimido-2 acétamido]-2 N-méthyl N-phényl-acétamide dans 60 cm³ de méthanol, on ajoute 1,3 g d'hydrate d'hydrazine. Le mélange réactionnel est agité à reflux pendant 30 minutes, après refroidissement on ajoute 100 cm³ d'eau. Le mélange est concentré à environ 100 cm³ puis amené à pH 9 avec une solution aqueuse de soude 2N et extrait par 2 fois 50 cm³ d'acétate d'éthyle. Les phases organiques réunies sont lavées par 2 fois 50 cm³ d'eau, séchées sur sulfate de magnésium puis concentrées à sec sous pression réduite (2,7 kPa) à 40°C. On obtient ainsi 3,0 g d'[amino-2 N-(méthoxy-3 phényl) acétamido]-2 N-méthyl N-phényl-acétamide sous forme d'une huile utilisée telle quelle dans les synthèses ultérieures.

Le [N-(méthoxy-3 phényl) phtalimido-2 acétamido]-2 N-méthyl N-phényl-acétamide peut être préparé de la manière suivante : à une suspension de 80,6 g d'acide [N-(méthoxy-3 phényl) phtalimido-2 acétamido]-2 acétique dans 900 cm³ de dichloro-1,2 éthane on ajoute 10 cm³ de diméthylformamide puis en 1 heure, 30,2 g de dichlorure d'oxalyle. Le mélange est agité 2 heures à une température voisine de 25°C, puis on ajoute 58,6 g de N-méthyl aniline en 45°minutes. Le mélange réactionnel est agité 2 heures à une température voisine de 25°C, lavé par 2 fois 500 cm³ d'eau puis 300 cm³ d'une solution aqueuse saturée d'hydrogénocarbonate de sodium, séché sur sulfate de magnésium et concentré à sec sous pression réduite (2,7 kPa) à 40°C. Le résidu est agité une heure dans 300 cm³ d'oxyde de diisopropyle, le produit insoluble est séparé par filtration, lavé par 3 fois 60 cm³ d'oxyde de diisopropyle et séché à l'air. On obtient ainsi, 84 g de [N-(méthoxy-3 phényl) phtalimido-2 acétamido]-2 N-méthyl N-phényl-acétamide fondant à 137°C.

L'acide [N-(méthoxy-3 phényl) phtalimido-2 acétamido]-2 acétique peut être préparé de la manière suivante : à une solution de 42,0 g de [N-(méthoxy-3 phényl) phtalimido-2 acétamido]-2 acétate de tert-butyle dans 500 cm³ de chlorure de méthylène, on ajoute 74,0 g d'acide trifluoroacétique. La solution obtenue est agitée à reflux pendant 5 heures, puis concentrée à sec sous pression réduite (2,7 kPa) à 40°C. Le résidu est agité pendant une heure dans 100 cm³ d'oxyde de diisopropyle, le produit insoluble est séparé par filtration, lavé par 3 fois 40 cm³ d'oxyde de diisopropyle et séché à l'air. On obtient ainsi, 36 g d'acide [N-(méthoxy-3 phényl) phtalimido-2 acétamido]-2 acétique fondant à 203°C.

Le [N-(méthoxy-3 phényl) phtalimido-2 acétamido]-2 acétate de tert-butyle peut être préparé de la manière suivante : à une solution de 96 g de N-(méthoxy-3 phényl) phtalimido-2 acétamide dans 1000 cm³ de tétrahydrofuranne anhydre, on ajoute en 30 minutes 14,9 g d'une suspension huileuse (60% en poids) d'hydrure de sodium. La suspension est agitée pendant 4 heures à une température voisine de 20°C puis on ajoute en 15 minutes 72,7 g de bromoacétate de tert-butyle. Le mélange réactionnel est agité pendant 16 heures à une température voisine de 25°C, hydrolysé lentement par 50 cm³ d'eau, puis concentré à sec sous pression réduite. Le résidu obtenu est agité pendant une heure dans 400 cm³ d'eau, le produit insoluble est séparé par filtration, lavé par 3 fois 100 cm³ d'eau, 2 fois 100 cm³ d'oxyde de diisopropyle et séché à l'air. On obtient ainsi, 82,0 g de [N-(méthoxy-3 phényl) phtalimido-2 acétamido]-2 acétate de tert-butyle fondant à 148°C.

Le N-(méthoxy-3 phényl) phtalimido-2 acétamide peut être préparé de la manière suivante : à une solution de 26,0 g de méthoxy-3 aniline dans 200 cm³ de chlorure de méthylène, on ajoute 22,0 g de triéthylamine puis 48,0 g de chlorure de phtalimido-2 acétyle en solution dans 300 cm³ de chlorure de méthylène en maintenant la température à environ 20°C. Le mélange réactionnel est agité pendant 4 heures, à cette température, puis on ajoute 800 cm³ d'eau. Le produit insoluble est séparé par filtration, lavé par 3 fois 100 cm³ d'eau et séché à l'air. On obtient ainsi 65,0 g de N-(méthoxy-3 phényl) phtalimido-2 acétamide fondant à 171°C.

Le chlorure de phtalimido-2 acétyle peut être préparé selon la méthode décrite par W. GRASSMANN et E. SCHULTE-UEBBING, Chem. Ber., 83, 244-247, (1950).

## Revendications

1. Les isomères lévogyres des (amino-3 phényl)-1 éthanesulfonate d'alkylammonium de formule : 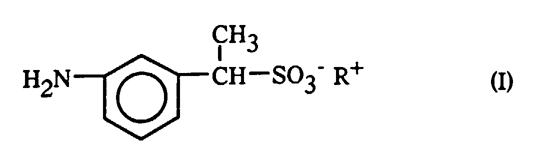 dans laquelle R⁺ représente un reste tétraalkylammonium ou trialkylphénylalkyl ammonium.

2. L'(amino-3 phényl)-1 éthanesulfonate de (tétra-n-butyl) ammonium (isomère lévogyre).

3. Procédé de préparation des composés de formule (I) selon la revendication 1 comprenant les étapes suivantes :
a) action de sulfite d'un métal alcalin sur le (bromo-1 éthyl)-1 nitro-3 benzène-(RS) et passage au sel de potassium pour obtenir le (nitro-3 phényl)-1 éthanesulfonate de potassium-(RS),
b) transformation du sel de potassium obtenu en a) en (nitro-3 phényl)-1 éthanesulfonate de benzylquininium mélange des formes A et B, séparation de la forme A et récupération d'un mélange enrichi en forme B,
c) conversion du sel de benzylquininium enrichi en forme B obtenu en b) en (nitro-3 phényl)-1 éthanesulfonate de potassium puis en (nitro-3 phényl)-1 éthanesulfonate de phénylglycinol forme B et enfin en (nitro-3 phényl)-1 éthanesulfonate de tétraalkylammonium ou trialkylphénylalkylammonium (isomère lévogyre),
d) réduction du produit obtenu en c) en composé de formule (I), isomère lévogyre.

4. Procédé selon la revendication 3 dans lequel l'étape a) est effectuée en solution aqueuse, à une température comprise entre 50 °C et 100°C.

5. Procédé selon la revendication 4 dans lequel la température est de 80°C.

6. Procédé selon l'une des revendications 3 à 5 pour lequel on utilise le sulfite de sodium ou de potassium.

7. Procédé selon l'une des revendications 4 à 6 dans lequel la récupération du produit s'effectue par l'intermédiaire d'un sel de tétraalkylammonium ou de trialkylphénylalkylammonium.

8. Procédé selon l'une des revendications 3 à 7 pour lequel l'étape b) s'effectue au moyen d'un halogénure de N-benzylquininium, en présence de dihydrogénophosphate de potassium, en milieu aqueux, à une température comprise entre 10°C et 30°C.

9. Procédé selon la revendication 8 pour lequel on utilise le chlorure de N-benzylquininium,

10. Procédé selon l'une des revendications 3 à 9 pour lequel le sel de potassium de l'étape c) est obtenu par action de nonafluorobutanesulfonate de potassium, au sein d'un solvant inerte, à une température comprise entre 10°C et 30°C.

11. Procédé selon l'une des revendications 3 à 10 pour lequel dans l'étape c) le sel de phénylglycinol est obtenu par action de (R)-(-)-phénylglycinol, en présence d'un acide, en milieu aqueux, à une température comprise entre 10 °C. et 30 °C.

12. Procédé selon l'une des revendications 3 à 11 pour lequel dans l'étape c) la transformation en sel d'alkylammonium ou de trialkylphénylalkylammonium s'effectue au moyen d'un sel de tétraalkylammonium ou de trialkylphénylalkylammonium, en milieu aqueux, à une température de 20°C.

13. Procédé selon la revendication 12 pour lequel on utilise un hydrogénosulfate de tétraalkylammonium ou de trialkylphénylalkylammonium.

14. Procédé selon l'une des revendications 3 à 13 pour lequel la réduction s'effectue sous pression d'hydrogène, en présence d'un catalyseur d'hydrogénation, au sein d'un solvant inerte et à une température voisine de 25°C.

15. Procédé selon la revendication 14 pour lequel on utilise une pression d'hydrogène de 100 kPa.

16. Procédé selon la revendication 3 pour la préparation de l'(amino-3 phényl)-1 éthanesulfonate de (tétra-n-butyl) ammonium, isomère lévogyre.

17. Application des composés selon la revendication 1 pour la préparation des énantiomères des composés de formule : 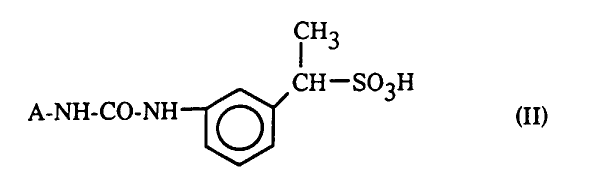 dans laquelle A représente
A) un reste -CH₂-CO-NR₁R₂ dans lequel R₁ représente un radical (a) phényle éventuellement substitué par un ou plusieurs substituants choisis parmi les atomes d'halogène et les radicaux alkyle, alcoxy, hydroxy polyfluoroalkyle, nitro, alkylthio, alcoxycarbonyle, carboxy, acylamino, méthylènedioxy, polyfluoroalcoxy, trifluorométhylthio, phénoxy, phényl, benzyle, phénylamino et CONR₃R₄, identiques ou différents, représentent un atome d'hydrogène, un radical alkyle, phényle (éventuellement substitué par un ou plusieurs substituants choisis parmi les atomes d'halogène et les radicaux alkyle, alcoxy et alkylthio), indanyle, cycloalkylalkyle, cycloalkyle, phénylalkyle ou bien R₃ et R₄ forment ensemble avec l'atome d'azote auquel ils sont rattachés un hétérocycle mono ou polycyclique saturé ou insaturé contenant 4 à 9 atomes de carbone et un ou plusieurs hétéroatomes (O,N,S) et éventuellement substitué par un plusieurs radicaux alkyle, alcoxy, alcoxycarbonyle, dialkylcarbamoyle, phényle ou en combinaison avec un atome de carbone de l'hétérocycle par un cycle spiromonocyclique à 4 ou 5 chaînons et contenant éventuellement un ou plusieurs hétéroatomes (O,S,N) ; b) pyridyle, c) isoquinolyle, d) quinolyle, e) quinoxalinyle (ces hétérocycles étant éventuellement substitués par un ou plusieurs substituants choisis parmi les radicaux alkyle, phényle et les atomes d'halogène), f) alkyle, g) phénylalkyle, h) naphtyle, i) tétrahydro-5,6,7,8 naphtyle, j) tétrahydro-1,2,3,4 naphtyle, k) alcoxycarbonylalkyle ou l) cycloalkyle. R₂ représente une chaîne -CH(R₅)-CO-R₆ dans laquelle R₅ représente un atome d'hydrogène ou un radical alkyle, alcoxycarbonyle ou phényle (éventuellement substitué par un ou plusieurs substituants choisis parmi les atomes d'halogène et les radicaux alkyle, alcoxy, alkylthio, nitro et amino) et R₆ représente un radical alcoxy, cycloalkyloxy (éventuellement substitué par au moins un radical alkyle), cycloalkylalkyloxy, phénylalkyloxy, polyfluoroalkyloxy cinnamyloxy et -NR₃R₄.
B) un reste de formule : 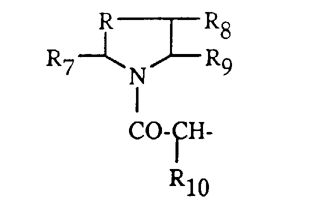 dans laquelle :
- soit R représente un radical méthylène, éthylène, SO, SO₂, CHOH ou un atome de soufre, R₇ représente un radical pyridyle éventuellement substitué par un ou plusieurs radicaux alkyle, furyle éventuellement substitué par un ou plusieurs radicaux alkyle, thiènyle éventuellement substitué par un ou plusieurs radicaux alkyle, quinolyle éventuellement substitué par un ou plusieurs radicaux alkyle, naphtyle éventuellement substitué par un ou plusieurs radicaux alkyle, indolyle éventuellement substitué par un ou plusieurs radicaux alkyle ou phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-NR₁₁R_{12,} -NH-CO-CH₃, trifluorométhyle ou trifluorométhoxy et R₈ représente un atome d'hydrogène,
- soit R représente un radical méthylène, R₇ représente un atome d'hydrogène et R₈ représente un radical phényle,
- soit R représente un radical CHR₁₃, R₇ et R₈ représentent chacun un atome d'hydrogène,
- R₉ représente un radical alcoxycarbonyle, cycloalkyloxycarbonyle, cycloalkylalkyloxycarbcnyle, -CONR₁₄R₁₅ ou phényle éventuellement substitué par un ou plusieurs substituants choisis parmi les radicaux alkyle, alcoxy ou hydroxy,
- R₁₀ représente un atome d'hydrogène ou un radical alkyle,
- R₁₃ représente un radical phényle,
- R₁₁ représente un atome d'hydrogène ou un radical alkyle, phénylalkyle ou phényle éventuellement substitué par un ou plusieurs substituants choisis parmi les atomes d'halogène et les radicaux alkyle, alcoxy et alkylthio,
- R₁₂ représente un radical alkyle, phénylalkyle ou phényle éventuellement substitué par un ou plusieurs substituants choisis parmi les atomes d'halogène et les radicaux alkyle, alcoxy et alkylthio,
ou bien R₁₁ et R₁₂ forment avec l'atome d'azote auquel ils sont rattachés un hétérocycle mono ou polycyclique saturé ou insaturé contenant 4 à 9 atomes de carbone et un ou plusieurs hétéroatomes (O, N) et éventuellement substitué par un ou plusieurs radicaux alkyle,
- R₁₄ représente un atome d'hydrogène ou un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
- R₁₅ représente un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
ou bien R₁₄ et R₁₅ forment ensemble avec l'atome d'azote auquel ils sont rattachés un hétérocycle mono ou polycyclique saturé ou insaturé contenant 4 à 9 atomes de carbone et un ou plusieurs hétéroatomes (O, N, S) et éventuellement substitué par un ou plusieurs radicaux alkyle,
C) un reste de formule : 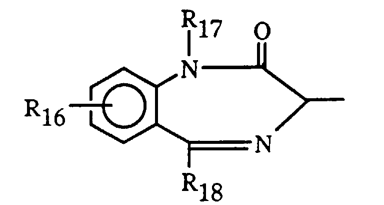 dans laquelle :
- R₁₆ représente un atome d'hydrogène ou d'halogène ou un radical alkyle, alkylthio, nitro, hydroxy ou cyano,
- R₁₇ représente un radical alkyle ou un reste -CH (R₅)-CO-R₆,
- R₁₈ représente un radical pyridyle ou phényle éventuellement substitué par un ou plusieurs substituants choisis parmi les atomes d'halogène et les radicaux alkyle, alcoxy, hydroxy, carboxy et nitro.

## Claims

1. The laevorotatory isomers of alkylammonium 1-(3-aminophenyl)ethanesulphonates of formula: 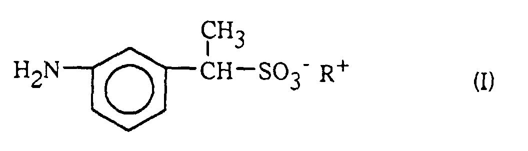 in which
R⁺ represents a tetraalkylammonium or trialkylphenylalkylammonium residue.

2. (Tetra-n-butyl)-ammonium 1-(3-aminophenyl)ethanesulphonate (laevorotatory isomer).

3. Process for the preparation of the compounds of formula (I) according to Claim 1, which comprises the following stages:
a) action of an alkali metal sulphite on (RS)-1-(1-bromoethyl)-3-nitrobenzene and conversion to the potassium salt to give potassium (RS)-1-(3-nitrophenyl)ethanesulphonate,
b) conversion of the potassium salt obtained in a) into a mixture of the A and B forms of benzylquininium 1-(3-nitrophenyl)ethanesulphonate, separation of the A form and recovery of a mixture enriched in the B form,
c) conversion of the benzylquininium salt, enriched in the B form, obtained in b) into potassium 1-(3-nitrophenyl)ethanesulphonate, thereafter into phenylglycinol 1-(3-nitrophenyl)ethanesulphonate, B form, and finally into tetraalkylammonium or trialkylphenylalkylammonium 1-(3-nitrophenyl)ethanesulphonate (laevorotatory isomer),
d) reduction of the product obtained in c) to give the compound of formula (I), laevorotatory isomer.

4. Process according to Claim 3, in which stage a) is carried out in aqueous solution at a temperature of between 50°C and 100°C.

5. Process according to Claim 4, in which the temperature is 80°C.

6. Process according to one of Claims 3 to 5, for which sodium sulphite or potassium sulphite is used.

7. Process according to one of Claims 4 to 6, in which the recovery of the product is effected via a tetraalkylammonium or trialkylphenylalkylammonium salt.

8. Process according to one of Claims 3 to 7, for which stage b) is effected by means of an N-benzylquininium halide, in the presence of potassium dihydrogenphosphate in an aqueous medium at a temperature of between 10°C and 30°C.

9. Process according to Claim 8, for which N-benzylquininium chloride is used.

10. Process according to one of Claims 3 to 9, for which the potassium salt of stage c) is obtained by the action of potassium nonafluorobutanesulphonate in an inert solvent at a temperature of between 10°C and 30°C.

11. Process according to one of Claims 3 to 10, for which, in stage c), the phenylglycinol salt is obtained by the action of (R)-(-)-phenylglycinol in the presence of an acid, in an aqueous medium, at a temperature of between 10°C and 30°C.

12. Process according to one of Claims 3 to 11, for which, in stage c), the conversion to an alkylammonium salt or trialkylphenylalkylammonium salt is effected by means of a tetraalkylammonium or trialkylphenylalkylammonium salt, in an aqueous medium, at a temperature of 20°C.

13. Process according to Claim 12, for which a tetraalkylammonium hydrogensulphate or trialkylphenylalkylammonium hydrogensulphate is used.

14. Process according to one of Claims 3 to 13, for which the reduction is effected under hydrogen pressure, in the presence of a hydrogenation catalyst, in an inert solvent and at a temperature of about 25°C.

15. Process according to Claim 14, for which a hydrogen pressure of 100 kPa is used.

16. Process according to Claim 3, for the preparation of the laevorotatory isomer of (tetra-n-butyl)ammonium 1-(3-aminophenyl)ethanesulphonate.

17. Application of the compounds according to Claim 1 for the preparation of the enantiomers of the compounds of formula: 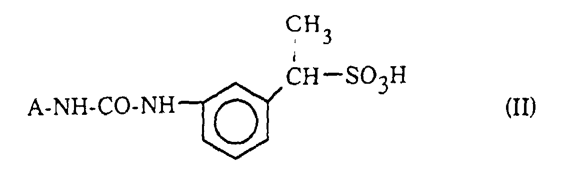 in which A represents
A) a -CH₂-CO-NR₁R₂ residue, in which R₁ represents one of the following radicals (a) phenyl optionally substituted by one or more substituents chosen from among halogen atoms and alkyl, alkoxy, hydroxyl polyfluoroalkyl, nitro, alkylthio, alkoxycarbonyl, carboxyl, acylamino, methylenedioxy, polyfluoroalkoxy, trifluoromethylthio, phenoxy, phenyl, benzyl, phenylamino and CONR₃R₄ radicals, which may be identical or different, represent a hydrogen atom or an alkyl, phenyl (optionally substituted by one or more substituents chosen from among halogen atoms and alkyl, alkoxy and alkylthio radicals), indanyl, cycloalkylalkyl, cycloalkyl or phenylalkyl radical or else R₃ and R₄ together with the nitrogen atom to which they are attached form a saturated or unsaturated monocyclic or polycyclic heterocyclic ring containing 4 to 9 carbon atoms and one or more hetero atoms (O, N or S) and optionally substituted by one or more alkyl, alkoxy, alkoxycarbonyl, dialkylcarbamoyl or phenyl radicals or, in combination with a carbon atom of the heterocyclic ring, by a spiromonocyclic ring having 4 or 5 members and optionally containing one or more hetero atoms (O, S or N); b) pyridyl, c) isoquinolyl, d) quinolyl, e) quinoxalinyl (these heterocyclic rings being optionally substituted by one or more substituents chosen from among alkyl and phenyl radicals and halogen atoms), f) alkyl, g) phenylalkyl, h) naphthyl, i) 5,6,7,8-tetrahydronaphthyl, j) 1,2,3,4-tetrahydronaphthyl, k) alkoxycarbonylalkyl or l) cycloalkyl.
R₂ represents a -CH(R₅)-CO-R₆ chain in which R₅ represents a hydrogen atom or an alkyl or alkoxycarbonyl radical or a phenyl radical (optionally substituted by one or more substituents chosen from among halogen atoms and alkyl, alkoxy, alkylthio, nitro and amino radicals), and R₆ represents an alkoxy, cycloalkoxy (optionally substituted by at least one alkyl radical), cycloalkylalkoxy, phenylalkoxy, polyfluoroalkoxy cinnamyloxy radical and -NR₃R₄.
B) a residue of formula: 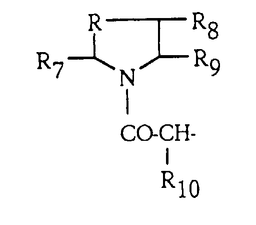 in which:
- either R represents a methylene, ethylene, SO, SO₂ or CHOH radical or a sulphur atom, R₇ represents a pyridyl radical optionally substituted by one or more alkyl radicals, a furyl radical optionally substituted by one or more alkyl radicals, a thienyl radical optionally substituted by one or more alkyl radicals, a quinolyl radical optionally substituted by one or more alkyl radicals, a naphthyl radical optionally substituted by one or more alkyl radicals, an indolyl radical optionally substituted by one or more alkyl radicals or a phenyl radical optionally substituted by one or more substituents chosen from among halogen atoms and alkyl, alkoxy, hydroxyl, nitro, amino, monoalkylamino, dialkylamino, alkoxycarbonyl, -CO-NR₁₁R₁₂, -NH-CO-CH₃, trifluoromethyl or trifluoromethoxy radicals and R₈ represents a hydrogen atom,
- or R represents a methylene radical, R₇ represents a hydrogen atom and R₈ represents a phenyl radical,
- or R represents a CHR₁₃ radical, and R₇ and R₈ each represent a hydrogen atom,
- R₉ represents an alkoxycarbonyl, cycloalkoxycarbonyl, cycloalkylalkoxycarbonyl or -CONR₁₄R₁₅ radical or a phenyl radical optionally substituted by one or more substituents chosen from among alkyl, alkoxy or hydroxyl radicals,
- R₁₀ represents a hydrogen atom or an alkyl radical,
- R₁₃ represents a phenyl radical,
- R₁₁ represents a hydrogen atom or one of the following radicals: alkyl, phenylalkyl or phenyl optionally substituted by one or more substituents chosen from among halogen atoms and alkyl, alkoxy and alkylthio radicals,
- R₁₂ represents one of the following radicals: alkyl, phenylalkyl or phenyl optionally substituted by one or more substituents chosen from among halogen atoms and alkyl, alkoxy and alkylthio radicals,
or else R₁₁ and R₁₂, together with the nitrogen atom to which they are attached, form a saturated or unsaturated monocyclic or polycyclic heterocyclic ring containing 4 to 9 carbon atoms and one or more hetero atoms (O or N) and optionally substituted by one or more alkyl radicals,
- R₁₄ represents a hydrogen atom or one of the following radicals: alkyl, cycloalkylalkyl, cycloalkyl, phenylalkyl or phenyl optionally substituted by one or more substituents chosen from among halogen atoms and alkyl, alkoxy and alkylthio radicals,
- R₁₅ represents one of the following radicals: alkyl, cycloalkylalkyl, cycloalkyl, phenylalkyl or phenyl optionally substituted by one or more substituents chosen from among halogen atoms and alkyl, alkoxy and alkylthio radicals,
or else R₁₄ and R₁₅, together with the nitrogen atom to which they are attached, form a saturated or unsaturated monocyclic or polycyclic heterocyclic ring containing 4 to 9 carbon atoms and one or more hetero atoms (O, N or S) and optionally substituted by one or more alkyl radicals,
C) a residue of formula: 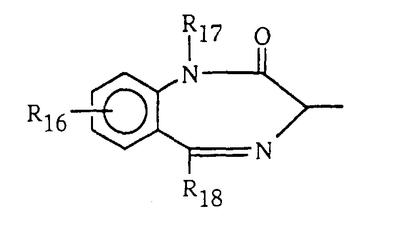 in which:
- R₁₆ represents a hydrogen or halogen atom or an alkyl, alkylthio, nitro, hydroxyl or cyano radical,
- R₁₇ represents an alkyl radical or a -CH(R₅)-CO-R₆ residue,
- R₁₈ represents a pyridyl radical or phenyl radical optionally substituted by one or more substituents chosen from among halogen atoms and alkyl, alkoxy, hydroxyl, carboxyl and nitro radicals.

## Patentansprüche

1. Die linksdrehenden Isomeren von Alkylammonium-1-(3-aminophenyl)-ethansulfonat der Formel 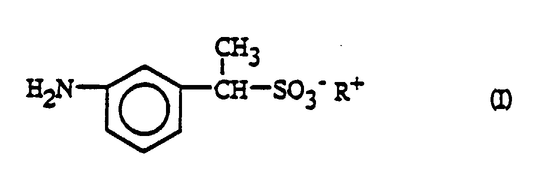 worin R⁺ einen Tetraalkylammonium- oder Trialkylphenylalkylammoniumrest darstellt.

2. Das (Tetra-n-butyl)-ammonium-1-(3-aminophenyl)-ethansulfonat (linksdrehendes Isomeres).

3. Verfahren zur Herstellung der Verbindungen der Formel (I) gemäß Anspruch 1, umfassend die folgenden Stufen:
a) Einwirkung eines Alkalimetallsulfits auf (RS)-1-(1-Bromoethyl)-3-nitrobenzol und Übergang zum Kaliumsalz, um das (RS)Kalium-1-(3-nitrophenyl)-ethansulfonat zu erhalten;
b) Umwandlung des in a) erhaltenen Kaiiumsalzes zum Benzylchininium-1-(3-nitrophenyl)-ethansulfonat, Gemisch der Formen A und B, Abtrennung der Form A und Gewinnung eines an Form B angereicherten Gemisches;
c) Umwandlung des in b) erhaltenen an Form B angereicherten Benzylchininiumsalzes zum Kalium-1-(3-nitrophenyl)-ethansulfonat, dann zum Phenylglycinol-1-(3-nitrophenyl)-ethansulfonat Form B, und schließlich zum Tetraalkylammonium- oder Trialkylphenylalkylammonium-1-(3-nitrophenyl)-ethansulfonat (linksdrehendes Isomeres),
d) Reduktion des in c) erhaltenen Produkts zur Verbindung der Formel (I), linksdrehendes Isomeres.

4. Verfahren gemäß Anspruch 3, worin die Stufe a) in wäßriger Lösung bei einer Temperatur zwischen 50 °C und 100 °C bewirkt wird.

5. Verfahren gemäß Anspruch 4, worin die Temperatur 80 °C beträgt.

6. Verfahren gemäß einem der Ansprüche 3 bis 5, wobei man das Natrium- oder Kaliumsulfit verwendet.

7. Verfahren gemäß einem der Ansprüche 4 bis 6, wobei die Gewinnung des Produkts über ein Tetraalkylammonium- oder Trialkylphenylalkylammoniumsalz bewirkt wird.

8. Verfahren gemäß einem der Ansprüche 3 bis 7, wobei die Stufe b) mittels eines N-Benzylchininiumhalogenids in Gegenwart von Kallumdihydrogenophosphat in wäßrigem Milieu bei einer Temperatur zwischen 10 °C und 30 °C bewirkt wird.

9. Verfahren gemäß Anspruch 8, wobei man N-Benzylchininiumchlorid verwendet.

10. Verfahren gemäß einem der Ansprüche 3 bis 9. worin das Kaliumsalz der Stufe c) erhalten wird durch Einwirkung von Kaliumnonafluorobutansulfonat in einem inerten Lösungsmittel bei einer Temperatur zwischen 10 °C und 30 °C.

11. Verfahren gemäß einem der Ansprüche 3 bis 10, wobei in der Stufe c) das Phenylglycinolsalz durch Einwirkung von (R)-(-)-Phenylgylcinol in Gegenwart einer Säure in wäßrigem Milieu bei einer Temperatur zwischen 10 °C und 30 °C erhalten wird.

12. Verfahren gemäß einem der Ansprüche 3 bis 11, wobei in der Stufe c) die Umwandlung zum Alkylammonium- oder Trialkylphenylalkylammoniumsalz mittels eines Tetraalkylammonium- oder Trialkylphenylalkylammoniumsalzes in wäßrigem Milieu bei einer Temperatur von 20 °C bewirkt wird.

13. Verfahren gemäß Anspruch 12, wobei man ein Tetraalkylammonium- oder Trialkylphenylalkylammoniumhydrogensulfat verwendet.

14. Verfahren gemäß einem der Ansprüche 3 bis 13, wobei die Reduktion unter Wasserstoffdruck in Gegenwart eines Hydrierungskatalysators in einem inerten Lösungsmittel und bei einer Temperatur nahe bei 25 °C bewirkt wird.

15. Verfahren gemäß Anspruch 14, wobei man einen Wasserstoffdruck von 100 kPa verwendet.

16. Verfahren gemäß Anspruch 3 zur Herstellung von (Tetra-n-butyl)-ammonium-1-(3-aminophenyl)-ethansulfonat, linksdrehendes Isomeres.

17. Anwendung der Verbindungen gemäß Anspruch 1 zur Herstellung der Enantiomeren der Verbindungen der Formel: 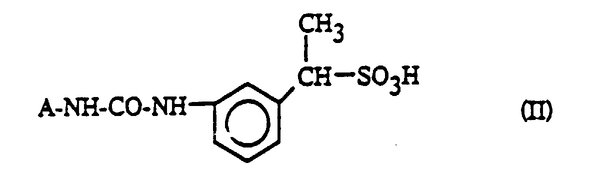 worin A bedeutet
A) einen Rest -CH₂-CO-NR₁R₂, worin R₁ bedeutet einen Rest
(a) Phenyl, gegebenenfalls substituiert durch einen mehrere Substituenten, ausgewählt unter den Halogenatomen und den Resten Alkyl, Alkoxy, Hydroxy, Polyfluoroalkyl, Nitro, Alkylthio, Alkoxycarbonyl, Carboxy, Acylamino, Methylendioxy, Polyfluoroalkoxy, Trifluoromethylthio, Phenoxy, Phenyl, Benzyl, Phenylamino und CONR₃R₄, die identisch oder verschieden sind, bedeuten ein Wasserstoffatom, einen Alkylrest, Phenyl (gegebenenfalls substituiert durch einen oder mehrere Substituenten, ausgewählt unter den Halogenatomen und den Resten Alkyl, Alkoxy und Alkylthio), Indanyl, Cycloalkylalkyl, Cycloalkyl, Phenylalkyl oder auch R₃ und R₄ bilden zusammen mit dem Stickstoffatom, an das sie gebunden sind, einen mono- oder polycyclischen Heterocyclus, der gesättigt oder ungesättigt ist, enthaltend 4 bis 9 Kohlenstoffatome und ein oder mehrere Heteroatome (O, N, S) und gegebenenfalls substituiert durch einen oder mehrere Rest(e) Alkyl, Alkoxy, Alkoxycarbonyl, Dialkylcarbamoyl, Phenyl oder in Kombination mit einem Kohlenstoffatom des Heterocyclus durch einen spiromonocyclischen Ring mit 4 oder 5 Kettengliedern und enthaltend gegebenenfalls ein oder mehrere Heteroatome (O, S, N);
(b) Pyridyl;
(c) Isochinolyl;
(d) Chinolyl;
(e) Chinoxalinyl (diese Heterocyclen sind gegebenenfalls substituiert durch einen oder mehrere Substituenten, ausgewählt unter den Resten Alkyl, Phenyl, und den Halogenatomen);
(f) Alkyl;
(g) Phenylalkyl;
(h) Naphthyl;
(i) 5,6,7,8-Tetrahydronaphthyl;
(j) 1,2,3,4-Tetrahydronaphthyl;
(k) Alkoxycarbonylalkyl, oder
(l) Cycloalkyl;
R₂ bedeutet eine Kette -CH(R₅)-CO-R₆, worin R₅ ein Wasserstoffatom oder einen Alkyl-, Alkoxycarbonyl- oder Phenylrest bedeuten (gegebenenfalls substituiert durch einen oder mehrere Substituenten, ausgewählt unter den Halogenatomen und den Resten Alkyl, Alkoxy, Alkylthio, Nitro und Amino) und
R₆ bedeutet einen Rest Alkoxy, Cycloalkoxy (gegebenenfalls substituiert durch mindestens einen Alkylrest), Cycloalkylalkyloxy, Phenylalkyloxy, Polyfluoroalkyloxy, Cinnamyloxy und -NR₃R₄;
B) einen Rest der Formel: 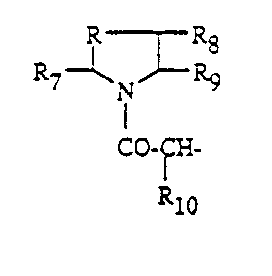 worin bedeuten:
- R entweder einen Methylen-, Ethylen-, SO-, SO₂-, CHOH-Rest oder ein Schwefelatom darstellt, R₇ bedeutet einen Pyridylrest, gegebenenfalls substituiert durch einen oder mehrere Alkylreste, Furyl, gegebenenfalls substituiert durch einen oder mehrere Alkylreste, Thienyl, gegebenenfalls substituiert durch einen oder mehrere Alkylreste, Chinolyl, gegebenenfalls substituiert durch einen oder mehrere Alkylreste, Naphthyl, gegebenenfalls substituiert durch einen oder mehrere Alkylreste, Indolyl, gegebenenfalls substituiert durch einen oder mehrere Alkylreste, oder Phenyl, gegebenenfalls substituiert durch einen oder mehrere Substituenten, ausgewählt unter den Halogenatomen und den Resten Alkyl, Alkoxy, Hydroxy, Nitro, Amino, Monoalkylamino, Dialkylamino, Alkoxycarbonyl, -CO-NR₁₁R₁₂, -NH-CO-CH₃, Trifluoromethyl oder Trifluoromethoxy, und R₈ bedeutet ein Wasserstoffatom,
- oder R bedeutet einen Methylenrest, R₇ bedeutet ein Wasserstoffatom, und R₈ bedeutet einen Phenylrest,
- oder R bedeutet einen Rest cHR₁₃, R₇ und R₈ bedeuten jeweils ein Wasserstoffatom,
- R₉ bedeutet einen Alkoxycarbonyl-, Cycloalkyloxycarbonyl-, Cycloalkylalkyloxycarbonyl-, -CONR₁₄R₁₅- oder Phenylrest, gegebenenfalls substituiert durch einen oder mehrere Substituenten, ausgewählt unter den Alkyl-, Alkoxy- oder Hydroxyresten,
- R₁₀ bedeutet ein Wasserstoffatom oder einen Alkylrest,
- R₁₃ bedeutet einen Phenylrest,
- R₁₁ bedeutet ein Wasserstoffatom oder einen Alkyl-, Phenylalkyl- oder Phenylrest, gegebenenfalls substituiert durch einen oder mehrere Substituenten, ausgewählt unter den Halogenatomen und den Alkyl-, Alkoxy- und Alkylthioresten,
- R₁₂ bedeutet einen Alkyl-, Phenylalkyl- oder Phenylrest, gegebenenfalls substituiert durch einen oder mehrere Substituenten, ausgewählt unter den Halogenatomen und den Alkyl-, Alkoxy- und Alkylthioresten,
oder aber R₁₁ und R₁₂ bilden zusammen mit dem Stickstoffatom, an das sie gebunden sind, einen gesättigten oder ungesättigten mono- oder polycyclischen Heterocyclus, enthaltend 4 bis 9 Kohlenstoffatome und ein oder mehrere Heteroatome (O, N) und gegebenenfalls substituiert durch einen oder mehrere Alkylreste,
R₁₄ bedeutet ein Wasserstoffatom oder einen Alkyl-, Cycloalkylalkyl-, Cycloalkyl-, Phenylalkyl- oder Phenylrest, gegebenenfalls substituiert durch einen oder mehrere Substituenten, ausgewählt unter den Halogenatomen und den Alkyl-, Alkoxy- und Alkylthioresten,
- R₁₅ bedeutet einen Alkyl-, Cycloalkylalkyl-, Cycloalkyl-, Phenylalkyl- oder Phenylrest, gegebenenfalls substituiert durch einen oder mehrere Substituenten, ausgewählt unter den Halogenatomen und den Alkyl-, Alkoxy- und Alkylthioressten,
oder aber R₁₄ und R₁₅ bilden zusammen mit dem Stickstoffatom, an das sie gebunden sind, einen gesättigten oder ungesättigten mono- oder polycyclischen Heterocyclus, enthaltend 4 bis 9 Kohlenstoffatome und ein oder mehrere Heteroatome (O, N, S), und gegebenenfalls substituiert durch einen oder mehrere Alkylreste,
C) einen Rest der Formel 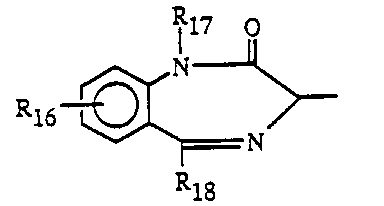 worin bedeuten:
- R₁₆ ein Wasserstoff- oder Halogenatom oder einen Alkyl-, Alkylthio-, Nitro-, Hydroxy- oder Cyanorest,
- R₁₇ einen Alkylrest oder einen Rest -CH(R₅)-CO-R₆,
- R₁₈ einen Pyridyl- oder Phenylrest, gegebenenfalls substituiert durch einen oder mehrere Substituenten, ausgewählt unter den Halogenatomen und den Alkyl-, Alkoxy-, Hydroxy-, Carboxy- und Nitroresten.
